# FASCICULE DE BREVET EUROPEEN

(11) **EP 3 195 734 B1**
(45) Date de publication et mention de la délivrance du brevet: **07.10.2020**
(21) Numéro de dépôt: 17305057.6
(22) Date de dépôt: 19.01.2017
(51) Int. Cl.: A23K 20/147, A61K 47/42, A61K 33/06, A23K 20/24, A23K 50/40, A61K 9/00, A61K 38/38

(54) **COMPLEMENT ALIMENTAIRE POUR UTILISATION DANS LA LUTTE CONTRE LE STRESS ET L'ANXIETE CHEZ LES ANIMAUX DE COMPAGNIE**
NAHRUNGSERGÄNZUNGSMITTEL FÜR DIE VERWENDUNG IM KAMPF GEGEN STRESS UND ÄNGSTE BEI HAUSTIEREN
FOOD SUPPLEMENT FOR USE IN COMBATING STRESS AND ANXIETY IN PETS

(30) Priorité: 21.01.2016 FR 1650472
(43) Date de publication de la demande: 26.07.2017
(73) Titulaire: Domes Pharma, 75014 Paris (FR)
(72) Inventeur: CHAUDER, Anne, 63000 CLERMONT-FERRAND (FR)
(74) Mandataire: Cabinet Laurent & Charras

(56) Documents cités:
- EP-A1- 2 110 028
- EP-A1- 2 218 462
- FR-A1- 2 704 392
- FR-A1- 2 723 680
- ANONYMOUS: "RESOURCES Feline Anti-Anxiety & Calming Formula (60 Tabs)", INTERNET CITATION, 9 mars 2004 (2004-03-09), page 1, XP002562403, Extrait de l'Internet: URL:http://www.entirelypets.com/resancalfo r.html [extrait le 2009-11-30]
- Geoffrey W Krissansen: "Emerging Health Properties of Whey Proteins and Their Clinical Implications INTRODUCTION AND BACKGROUND", Journal of the American College of Nutrition, 17 septembre 2007 (2007-09-17), pages 713-723, XP055109179, Extrait de l'Internet: URL:http://www.leadingthewhey.com/sites/de fault/files/health-properties-of-whey-and- clinical-implications.pdf [extrait le 2014-03-20]

## Description

La présente invention concerne une composition vétérinaire pour lutter contre le stress et l'anxiété chez les animaux de compagnie. Plus particulièrement, elle concerne un complément alimentaire destiné aux chats, aux chiens et aux NAC (nouveaux animaux de compagnie) en vue de calmer l'anxiété et les états de stress modérés chez ces animaux.

Chez les animaux de compagnie, en particulier les chats, les chiens et les NAC, il est assez fréquent de rencontrer un état de stress ou d'anxiété chronique ou passager.

Ce stress ou cette anxiété peuvent avoir différentes origines et facteurs déclenchant. Ils sont par exemple souvent liés à la solitude et aux conditions de captivité de l'animal qui peut manquer d'activité ou de stimulation (animaux laissés seuls, gardés en appartement, enfermés ou attachés), à des changements d'habitude ou à une modification du cadre de vie (arrivée ou départ d'une personne ou d'un animal, déménagement, travaux ou changement de mobilier ou de décoration...), à une séparation d'avec son maître, à des phobies ou des peurs liées à une situation inhabituelle (bruits stridents, orage, feux d'artifices, transport en voiture, visite chez le vétérinaire...).

Un tel état de stress ou d'anxiété peut se manifester de différentes façons selon les animaux, et notamment par des troubles du comportement et/ou des manifestations cliniques. On peut citer à titre d'exemple les aboiements ou miaulements excessifs ; l'hyperactivité et l'agressivité ; la malpropreté urinaire ou fécale ; le léchage excessif et compulsif ; le halètement, la transpiration et les tremblements ; la diarrhée et les vomissements ; la destruction de l'environnement et l' automutilation.

De tels comportements sont souvent difficiles à supporter pour les maîtres et peuvent être la cause d'une rapide dégradation des relations entre l'animal de compagnie et son maître. En outre, le stress et l'anxiété peuvent à la longue causer des problèmes de santé chez l'animal aux conséquences pouvant être graves, tels que des troubles gastro-entériques, dermatologiques ou urinaires, une prédisposition aux infections, de l'anorexie ou de la boulimie...

EP2218462A1 divulgue une préparation pharmaceutique pour le traitement de pathologies liées à la dépression, comprenant de l'a-lactalbumine.

Le but de l'invention est de proposer un complément alimentaire efficace pour combattre le stress modéré et l'état d'anxiété chez les animaux de compagnie et notamment chez les chats, les chiens et les NAC, tels que par exemples les lapins, hamsters, cochons d'inde, rats, souris et autres rongeurs, les furets...

Pour résoudre ce problème technique l'invention, enseigne un complément alimentaire à usage vétérinaire, destiné à lutter contre le stress et l'anxiété chez les animaux de compagnie, qui se caractérise en ce qu'il contient un mélange d'a-lactalbumine et de magnésium.

De préférence, le complément alimentaire selon l'invention contient du magnésium sous la forme de glycérophosphate de magnésium et/ou de stéarate de magnésium.

Selon l'invention, le complément alimentaire contient majoritairement, en tant que source de magnésium, du glycérophosphate de magnésium. En effet, les inventeurs ont remarqué, que le glycérophosphate de magnésium présentait une forte biodisponibilité et un goût moins amer que les autres formes de magnésium. En pratique, le glycérophosphate de magnésium représente au moins 80%, avantageusement plus de 90% en poids de la source de magnésium.

Selon l'invention, le complément alimentaire contient plus de 50 %, et de préférence environ 70 % en poids de mélange d'a-lactalbumine et de magnésium. Dans ce mélange, le ratio α-lactalbumine / magnésium est compris entre 50/50 et 80/20, de préférence de l'ordre de 70/30.

Selon un mode de réalisation préférentiel de l'invention, le complément alimentaire contient en outre au moins un excipient appétent.

La nature de cet excipient appétent est préférentiellement choisie en fonction de l'espèce animale à laquelle le complément alimentaire est destiné. Elle peut ainsi varier selon les applications envisagées. Il s'agit notamment de dérivés de sous-produits animaux.

Selon un mode de réalisation de l'invention, le complément alimentaire comprend en outre un ou plusieurs composés ou excipients choisis parmi les composés liants, les composés absorbants, les agents de suspension, les diluants, les excipients de compression, les dérivés cellulosiques, la cellulose microcristalline et le phosphate de calcium.

Selon un mode de réalisation de l'invention, le dosage utilisé est compris entre 15 mg et 60 mg de complément alimentaire par kilo de poids de l'animal concerné. Il est préférentiellement de 25 mg ou de 50 mg de complément alimentaire par kilo de poids de l'animal concerné.

Le complément alimentaire est destiné à être administré oralement. Pour cela, il peut se présenter sous différentes formes galéniques, par exemple sous forme de comprimés, d'une bouchée tendre, d'une poudre conditionnée en gélules, d'un liquide à verser sur la nourriture ou dans l'eau de boisson ou toute autre forme appropriée.

Selon un mode de réalisation préférentiel de l'invention, le complément alimentaire se présente sous la forme d'une poudre conditionnée en gélule. La poudre est destinée à être disperser sur l'alimentation sèche ou humide de l'animal pour ensuite être ingérée.

La posologie conseillée est par exemple d'une gélule par jour pour 5 kg de poids de l'animal. Le complément alimentaire est préférentiellement prévu pour être administré en cure d'un mois, éventuellement renouvelable. Il peut ainsi avantageusement être commercialisé en boîte de 30 gélules, ce qui correspond à la quantité nécessaire pour une cure d'un mois pour un chat moyen ou un petit chien.

Le complément alimentaire selon l'invention est efficace et présente une action calmante sans générer d'effets secondaires notables contrairement aux médicaments antidépresseurs classiques souvent responsables d'accoutumance et de somnolence.

En effet, en cas de stress, la sérotonine présente dans le cortex des individus est consommée afin de produire des protéines permettant de retourner à un état serein. Les animaux qui présentent un état d'anxiété ou de stress chronique ont une faible concentration de sérotonine dans le cortex due à une consommation en continu de celle-ci, ce qui induit un manque constant de sérotonine dans l'organisme et empêche un retour à la normale.

Or, la sérotonine ne passant pas la barrière intestinale, il n'est pas possible de combler ce manque par un apport direct de sérotonine dans l'alimentation des animaux stressés.

Dans l'organisme la sérotonine est fabriquée en plusieurs étapes à partir d'un acide aminé appelé tryptophane par des neurones spécifiques du tronc cérébral. Un apport en tryptophane par l'alimentation peut permettre l'augmentation de la concentration en sérotonine dans le cerveau.

Cependant, pour que le tryptophane arrive en quantité suffisante au niveau du cerveau, il faut surmonter de nombreuses difficultés : l'apport alimentaire doit être réalisé en quantité suffisante ; l'assimilation intestinale doit être satisfaisante ; le tryptophane ne doit pas être détourné vers d'autres voies et doit enfin être absorbé au niveau du cerveau.

Tout ceci nécessite un subtil équilibre d'apport des différents acides aminés.

L'a-lactalbumine contenue dans le complément alimentaire selon l'invention est une protéine alimentaire très riche en tryptophane (elle en contient environ 6%). Son apport, au moyen du complément alimentaire de l'invention, dans l'alimentation des animaux concernés permet d'augmenter le ratio tryptophane/grands acides aminés neutres. De cette façon, le tryptophane est plus avantagé dans la compétition pour atteindre le cerveau et la sécrétion de sérotonine est favorisée.

En outre, il a été démontré qu'un manque de magnésium mène à une augmentation des comportements anxieux et dépressifs chez les animaux. Le magnésium contenu dans le complément alimentaire selon l'invention, qui est un inhibiteur inorganique des récepteurs NMDA (N-méthyl-D-aspartate), présente une activité anxiolytique et comparable à un antidépresseur qui potentialise l'activité de l'a-lactalbumine par un effet de synergie.

L'invention et les avantages qui en découlent ressortiront bien de l'exemple suivant.

### 1/ Animaux et groupes expérimentaux

Quarante rats mâles Wistar Han (Crl :WI(Han), Charles River Laboratories, L'Arbresle, France), d'un poids de 250 à 275 g ont été utilisé. A la réception, les rats ont été marqués et répartis par groupes de 3 ou 4 animaux dans des cages en polycarbonate (Eurostandard type IIIH, 425 x 266 x 185 mm, Tecniplast, Lyon, France). Les animaux ont été stabulés dans une animalerie climatisée, à une température de 22 ± 2°C et une humidité relative de 50 ± 20 %. Les rats ont disposé de nourriture (Teklad Global 2016, Harlan, Ganat, France) et de boisson ad libitum et ont été maintenus dans un cycle lumière-obscurité inversé de 12 heures (lumière de 20h00 à 8h00).

Après une familiarisation d'une semaine aux conditions du laboratoire, les rats ont été pesés et répartis au hasard en 4 groupes de traitements (n = 10 / groupe) :
- Groupe 1 (Véhicule) : eau de source administrée p.o. 60 minutes (min) avant le test ;
- Groupe 2 (DZ) : Diazépam administré à la dose de 3 mg/kg p.o. 60 min avant le test ;
- Groupe 3 (I-25) : Produit Invention administré à la dose de 25 mg/kg p.o. 60 min avant le test ;
- Groupe 4 (I-50) : Produit invention administré à la dose de 50 mg/kg p.o. 60 min avant le test.

Afin d'éviter les interférences éventuelles entre les divers traitements, les rats d'une même cage ont tous reçu le même traitement à la même dose. Les rats des différents groupes ont tous été manipulés de manière identique et dans les mêmes conditions.

### 2/ Produits à tester et véhicule

Les produits I-25 et I-50 correspondent au complément alimentaire de l'invention contenant de l'a-lactalbumine (130 mg) et du glycérophosphate de magnésium (50 mg). Les produits ont été mis en solution dans de l'eau de source (Cristaline, Metzeral, France) à des concentrations de 2.5, 5 et 10 mg/ml. L'eau de source a également servi de traitement pour le groupe Véhicule.

Le diazépam sous forme de solution buvable (Valium 1%, Roche, Boulogne-Billancourt, France ; N° Lot : M0970M1-12/2013) a été mis en suspension dans une solution aqueuse contenant 0.5% méthylcellulose (Tylose® MH 300, Sigma-aldrich, St Quentin Fallavier, France) à une concentration de 0.3 mg/ml.

Les traitements ont été préparés extemporanément et administrés à un volume de 10 ml/kg.

### 3/ Etude des effets anxiolytiques du produit I administré par voie orale aux doses de 25 et 50 mg/kg, dans le test de l'EDC chez le rat mâle Wistar adulte

Le test s'est déroulé en 2 phases réparties sur 3 jours (J) :
- une phase d'habituation aux conditions du test (J1 et J2)
- une phase de test (J3).

Les rats ont reçu leur traitement à J3, le jour du test d'anxiété. L'ensemble des procédures a été réalisé en aveugle.

### 3.1 - Administration des traitements

A J3, chaque rat a reçu son traitement par voie orale à l'aide d'une seringue connectée à une sonde de gavage intragastrique (Popper & sons, inc., New York, USA) 60 min avant d'être testé dans le dispositif de l'EDC.

| **Groupe** | **Nb de rats** | **Produit testé** | **Dose** (mg/kg, p.o.) | **Volume** (ml/kg) | **Heure d'administrat ion** (min avant le test) |
|---|---|---|---|---|---|
| Véhicule | 10 | Eau de source | - | 10 | 60 |
| Diazépam | 10 | Diazépam + méthycellulose 0.5% | 3 | 10 | 60 |
| I-25 | 10 | I-25+ Eau de source | 25 | 10 | 60 |
| I-50 | 10 | I-50 + Eau de source | 50 | 10 | 60 |

### 3.2 - Test de l'EDC

### Dispositif expérimental

Le dispositif de l'EDC est composé d'une boîte en polycarbonate transparente (44 x 28 x 18 cm), dont le fond a été tapissé d'une couche de sciure de 5 cm d'épaisseur. Un orifice permettant la fixation d'une sonde électrique deux centimètres au-dessus du niveau de la sciure est présent au milieu de l'une des faces latérales. La sonde électrique est faite d'un bloc de Plexiglas (7 x 2 x 0,5 cm) recouvert de fils de cuivre. La sonde est reliée à un générateur de chocs électriques à déclenchement manuel (OPEN-Systems, Maxéville, France).

Le dispositif expérimental a été placé dans une pièce éclairée par une lumière rouge de faible intensité. Une caméra vidéo reliée à un enregistreur numérique a permis de sauvegarder l'ensemble des séquences comportementales afin de mesurer les comportements d'intérêts ultérieurement. Un moniteur de contrôle placé dans une pièce adjacente a permis à un opérateur d'administrer le choc électrique à chaque animal et de contrôler le déroulement du test.

### Habituation aux conditions du test

Les 2 jours précédant le test (J1 et J2), les rats d'une même cage ont été transportés de l'animalerie jusque dans la pièce d'expérimentation et placés ensemble dans le dispositif expérimental sans électrode pendant 20 minutes. Entre l'habituation de chaque cage de rat, la sciure a été changée et égalisée à une hauteur uniforme de 5 cm. Les animaux ont ensuite été ramenés à l'animalerie.

### Test d'anxiété

Le test d'anxiété s'est déroulé au cours des premières heures de la phase d'obscurité du cycle lumineux correspondant à la période d'activité maximale des animaux.

La sonde électrique a été insérée dans la cage avant le début du test. Chaque rat a été placé individuellement dans le dispositif expérimental du côté opposé à la sonde. Un seul et unique choc électrique de faible intensité (2 mA) a été délivré à l'animal au moment où il a posé ses pattes antérieures pour la première fois sur la sonde. Suite au choc électrique, le comportement du rat a été enregistré pendant 5 min. La sciure a été égalisée à une hauteur uniforme de 5 cm avant le passage de chaque rat et changée avant la session de test de chaque nouvelle cage de rats pour éviter les interférences éventuelles entre les groupes.

### Variables comportementales, étudiées

Les comportements suivants ont été quantifiés à partir des enregistrements vidéo réalisés au cours des tests :
- durée d'enfouissement de la sonde ;
- nombre d'étirements en direction de la sonde ;
- nombre d'approches vis-à-vis de la sonde ;
- nombre de fuites vis-à-vis de la sonde.

Les variables approches et fuites ont permis de calculer le pourcentage d'approches suivies de fuites : (nombre de fuites/nombre d'approches) x 100. Les variables "durée d'enfouissement", "nombre d'étirements" et "pourcentage d'approches suivies de fuites" ont servi à établir le score global d'anxiété.

Pour cela, un rang a été attribué à chaque rat sur chacune des 3 variables prises en compte. Pour l'attribution des rangs sur une variable donnée, les rats ont été classés par ordre croissant sur la base de cette variable. L'animal avec la valeur la plus faible a reçu le rang « 1 », l'animal suivant le rang « 2 », et ainsi de suite jusqu'à l'animal avec la valeur la plus élevée qui a reçu le rang « N » égal au nombre total de rats inclus dans l'étude. En cas d'égalité entre plusieurs rats, chacun de ces rats a reçu le rang moyen de la série de rats concernés (par exemple, si les 5 rats avec les valeurs les plus faibles sont à égalité - ils occupent collectivement les rangs de 1 à 5 - chacun de ces rats a reçu le rang moyen de la série : (1+2+3+4+5)/5 = 3).

Le score global d'anxiété de chaque rat correspond à la somme des rangs qui lui ont été attribués sur les 3 variables prises en comptes.

### 3.3 - Suivi des animaux et euthanasie

Les animaux ont été pesés 4 jours après leur arrivée au laboratoire, ainsi que le jour du test (J3). Le bien-être et la viabilité des animaux ont été vérifiés quotidiennement pendant toute la durée de l'étude. Les animaux ont été euthanasiés à la fin de l'étude par l'injection intrapéritonéale d'une surdose (110 mg) de pentobarbital sodique (Ceva Santé Animale, Libourne, France).

### 3.4 - Analyse statistique

L'effet des traitements sur le score global d'anxiété des rats a été analysé à l'aide du test de Kruskal-Wallis, suivi du test U de Mann-Whitney pour les comparaisons multiples. Le seuil de significativité a été fixé à 5%.

Les traitements statistiques et graphiques ont été réalisés à l'aide des logiciels Statview® 5 (SAS Institute, Inc., USA).

### 4 - Résultats

Le test de Kruskal-Wallis a montré une hétérogénéité significative parmi les scores globaux d'anxiété des rats des différents groupes de traitements [H(ddl=4)= 13.069 ; P = 0.009].

Le test de Mann-Whitney a montré que les scores globaux d'anxiété des rats des groupes Diazépam, I-25 et I-50 ont été significativement inférieurs à celui des rats du groupe véhicule.

| **Groupe** | **Véhicule** | **Diazépam** | **I-25** | **I-50** |
|---|---|---|---|---|
| | (*n* = 10) | (*n* = 9) | (*n* = 10) | (*n* = 10) |
| Médiane | 111 | 41 | 55 | 46 |
| QI - QS | 69 - 124 | 41 - 80 | 41 - 80 | 41 - 54 |
| Test de Mann-Whitney (vs.Véhicule) | | U = 12 | U = 23 | U = 15 |
| | | ***P* = 0.006** | ***P* = 0.041** | ***P* = 0.008** |

## Revendications

1. Complément alimentaire pour utilisation dans la lutte contre le stress et l'anxiété chez les animaux de compagnie, ledit complément alimentaire contenant un mélange d'a-lactalbumine et de magnésium, **caractérisé en ce que** ledit complément alimentaire contient plus de 50 % en poids de mélange d'a-lactalbumine et de magnésium, dans lequel le ratio α-lactalbumine / magnésium est compris entre 50/50 et 80/20, et **en ce qu'**il contient, en tant que source de magnésium, du glycérophosphate de magnésium représentant au moins 80% en poids de ladite source de magnésium.

2. Complément alimentaire pour utilisation selon la revendication 1, **caractérisé en ce qu'**il contient du magnésium sous la forme de stéarate de magnésium.

3. Complément alimentaire pour utilisation selon la revendication 1 ou 2, **caractérisé en ce que** le glycérophosphate de magnésium représente plus de 90% en poids de la source de magnésium.

4. Complément alimentaire pour utilisation selon l'une quelconque des revendications précédentes, **caractérisé en ce qu'**il contient environ 70 % en poids de mélange d'a-lactalbumine et de magnésium.

5. Complément alimentaire pour utilisation selon la revendication 4, **caractérisé en ce que** le ratio α-lactalbumine / magnésium est de l'ordre de 70/30.

6. Complément alimentaire pour utilisation selon l'une quelconque des revendications précédentes, **caractérisé en ce que** qu'il contient en outre au moins un excipient appétent.

7. Complément alimentaire pour utilisation selon l'une quelconque des revendications précédentes **caractérisé en ce qu'**il contient en outre un ou plusieurs composés ou excipients choisis parmi les composés liants, les composés absorbants, les agents de suspension, les diluants, les excipients de compression, les dérivés cellulosiques, la cellulose microcristalline et le phosphate de calcium.

8. Complément alimentaire pour utilisation selon l'une quelconque des revendications précédentes, **caractérisé en ce qu'**il se présente sous la forme de comprimés, d'une bouchée tendre (« soft chew »), d'une poudre conditionnée en gélules, d'un liquide à verser sur la nourriture ou dans l'eau de boisson

9. Complément alimentaire pour utilisation selon l'une quelconque des revendications précédentes, **caractérisé en ce qu'**il est à usage oral.

## Patentansprüche

1. Nahrungsergänzungsmittel für die Verwendung im Kampf gegen Stress und Ängste bei Haustieren, dieses Nahrungsergänzungsmittel enthält eine Mischung aus α-Lactalbumin und Magnesium, **dadurch gekennzeichnet, dass** dieses Nahrungsergänzungsmittel über 50 Gewichts-% einer Mischung aus α-Lactalbumin und Magnesium enthält, dass das Verhältnis α-Lactalbumin / Magnesium zwischen 50/50 und 80/20, liegt und dadurch, dass es als Magnesiumquelle Magnesiumglycerophosphat enthält, das mindestens 80 Gewichts-% dieser Magnesiumquelle darstellt.

2. Nahrungsergänzungsmittel für die Verwendung nach Anspruch 1, **dadurch gekennzeichnet, dass** es Magnesium in Form von Magnesiumustearat enthält.

3. Nahrungsergänzungsmittel für die Verwendung nach Anspruch 1 oder 2, **dadurch gekennzeichnet, dass** das Magnesiumglycerophosphat über 90 Gewichts-% der Magnesiumquelle darstellt.

4. Nahrungsergänzungsmittel für die Verwendung nach irgendeinem der vorangehenden Ansprüche, **dadurch gekennzeichnet, dass** es ungefähr 70 Gewichts-% der Mischung aus α-Lactalbumin und Magnesium enthält.

5. Nahrungsergänzungsmittel für die Verwendung nach Anspruch 4, **dadurch gekennzeichnet, dass** das Verhältnis α-Lactalbumin / Magnesium in einer Größneordnung von 70/30 liegt.

6. Nahrungsergänzungsmittel für die Verwendung nach irgendeinem der vorangehenden Ansprüche, **dadurch gekennzeichnet, dass** es außerdem mindestens einen Geschmacksstoff enthält.

7. Nahrungsergänzungsmittel für die Verwendung nach irgendeinem der vorangehenden Ansprüche, **dadurch gekennzeichnet, dass** es außerdem eine oder mehrere Verbindungen oder Hilfsstoffe enthält, die ausgewählt werden aus absorbierenden Verbindungen, suspendierenden Wirkstoffen, Lösungsmitteln, Kompressionshilfstoffen, Zellulosederivaten, mikrokristalliner Zellulose und Calciumphosphat.

8. Nahrungsergänzungsmittel für die Verwendung nach irgendeinem der vorangehenden Ansprüche, **dadurch gekennzeichnet, dass** es die Form von Tabletten, weicher Häppchen («soft chew »), eines in Kapseln verpackten Pulvers oder einer Flüssigkeit, die in die Nahrung oder das Trinkwasser gegeben wird, haben kann.

9. Nahrungsergänzungsmittel für die Verwendung nach irgendeinem der vorangehenden Ansprüche, **dadurch gekennzeichnet, dass** es zur oralen Verwendung bestimmt ist.

## Claims

1. Food supplement for use in the fight against stress and anxiety in pets, said food supplement containing a mixture of α-lactalbumin and magnesium, **characterized in that** said food supplement contains more than 50% by weight of the mixture of α-lactalbumin and magnesium, wherein the α-lactalbumin / magnesium ratio is between 50/50 and 80/20, and **in that** its contains, as a source of magnesium, magnesium glycerophosphate representing at least 80% by weight of the magnesium source.

2. Food supplement for its use according to claim 1, **characterized in that** it contains magnesium in the form of magnesium stearate.

3. Food supplement for its use according to claim 1 or 2, **characterized in that** the magnesium glycerophosphate represents more than 90% by weight of the magnesium source.

4. Food supplement for its use according to any one of the preceding claims, **characterized in that** it contains approximately 70% by weight of a mixture of α-lactalbumin and magnesium.

5. Food supplement for its use according to claim 4, **characterized in that** the α-lactalbumin / magnesium ratio is of the order of 70/30.

6. Food supplement for its use according to any one of the preceding claims, **characterized in that** it also contains at least one palatable excipient.

7. Food supplement for its use according to any one of the preceding claims, **characterized in that** it also contains one or more compounds or excipients chosen from binding compounds, absorbent compounds, suspending agents, diluents, compression excipients, cellulosic derivatives, microcrystalline cellulose and calcium phosphate.

8. Food supplement for its use according to any one of the preceding claims, **characterized in that** it is in the form of tablets, of a tender bite ("soft chew"), of a powder packaged in capsules, of a liquid to be poured on food or in drinking water.

9. Food supplement for its use according to any one of the preceding claims, **characterized in that** it is for oral use.
